(19) Europäisches Patentamt European Patent Office Office européen des brevets

(11) **EP 4 763 965 A1**

(12) **EUROPEAN PATENT APPLICATION**

(43) Date of publication:
**24.06.2026 Bulletin 2026/26**

(21) Application number: **24222448.3**

(22) Date of filing: **20.12.2024**

(51) International Patent Classification (IPC):
***C12M 1/00*** *(2006.01)* ***C12M 1/34*** *(2006.01)*

(52) Cooperative Patent Classification (CPC):
**C12M 29/18; C12M 41/00;** C12M 47/02

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **GEA TDS GmbH**
**31157 Sarstedt (DE)**

(72) Inventors:
• **Krampitz, Tatjana**
  **31157 Sarstedt (DE)**
• **Göhmann, Rüdiger**
  **59302 Oelde (DE)**
• **Vorländer, David**
  **31157 Sarstedt (DE)**
• **Bode, Sebastian**
  **31157 Sarstedt (DE)**

(74) Representative: **AWA Denmark A/S**
**Strandgade 56**
**1401 Copenhagen K (DK)**

(54) **PERFUSION SYSTEM WITH FLOW CONTROL**

(57)     The present invention relates to an apparatus for use in a biotechnological process, the apparatus comprising a vessel and a separator. The present invention also relates to a method for controlling flow of cells and culture medium between a vessel and a separator.

FIG. 2

## Description

## Field of Invention

The present invention relates to an apparatus for use in a biotechnological process, the apparatus comprising a vessel and a separator. The present invention also relates to a method for controlling flow of cells and culture medium between a vessel and a separator.

## Background of Invention

During cultivations, especially for cell cultures and/or those which are considered longer term cultivations, the spent cultivation medium must be removed and replaced by fresh media. To achieve this a solid-liquid separation process is typically coupled with a bioreactor in which the cultivation is taking place. The transfer from the bioreactor to the separation device as well as the return of concentrated cells from the separation device to the bioreactor must be controlled during the process runtime.

An example of a known system is disclosed in EP2020433, which relates to a continuous perfusion bioreactor system. The system disclosed herein comprises a first apparatus comprising a first collapsible bag adapted for containing a fluid and a second apparatus, wherein the second apparatus can form at least part of a separation device.

Within the apparatus used in such systems and during transfer between bioreactor and separator the cells must be handled carefully and shear stress which would kill the cells must be avoided or at least minimised. An example of a means to achieve this can be seen in WO2005/042768, which discloses a method for maintaining low shear in a bioprocessing system in which a peristaltic pump is used to limit the extent of cell damage on transfer of cells from a vessel to a separator.

In conventional systems there is at least one element in the flow path of the cells that contributes to shear stress on the cells, such as control valves, pinch valves or pumps. Due to this stress, the cell viability and therefore the possible yield is reduced. In a worst-case scenario, the perfusion process kills the cells faster than they are able to reproduce.

There is therefore a need for an apparatus and process which can reduce or even eliminate the impact of this, whilst still facilitating control of the flow between a vessel, such as a bioreactor, and a separator.

The present invention has been made from a consideration of this.

## Summary of Invention

Thus, according to a first aspect of the present invention, there is provided an apparatus for use in a biotechnological process, the apparatus comprising:

a vessel, the vessel being a bioreactor or storage tank, the vessel comprising a vessel inlet and a vessel outlet,
a separator, the separator comprising a separator inlet, a first separator outlet and a second separator outlet,
there being a first conduit from the vessel outlet to the separator inlet through which cells and culture medium can pass,
there being a second conduit from the first separator outlet to the vessel inlet through which cells and optionally culture medium can pass following a separation process,
characterized in that the apparatus further comprises a vertical-positioning adjuster configured to change the position of the vessel and/or the separator relative to a vertical axis during the course of the biotechnological process, preferably the vertical-positioning adjuster is configured to change the position of the separator relative to a vertical axis during the course of the biotechnological process.

The presence of the vertical-positioning adjuster configured to change the position of the vessel and/or the separator relative to a vertical axis during the course of the biotechnological process provides a means by which the flow of cells and culture medium between the vessel and the separator can be controlled. As a result, the reliance on components such as pumps and/or pinch valves which control flow in a way that requires a reduction in cross sectional area of the flow path is reduced or even removed entirely. This is beneficial as the shear stress to which the cells are exposed as a result of use of components such as pinch valves and pumps can be reduced, thereby reducing damage to cells and in turn providing greater yield in the process.

The apparatus of the invention provides means for perfusion which can reduce cell damage compared with conventional systems. In the perfusion process, spent culture medium and/or culture medium enriched with inhibiting metabolites, for example lactate or ammonia, is removed and according to the invention this is achieved whilst limiting damage to the cells. The removed culture medium can be replaced with fresh medium to maintain the inhibitor concentrations below critical levels. The apparatus which facilitates this in a way that provides reduced cell damage compared with conventional systems is advantageous, as reduced cell loss and therefore increased yields can be achieved.

In some embodiments, the vertical-positioning adjuster may comprise an electric or hydraulic position changing mechanism. These configurations for adjusting the vertical position are particularly beneficial as they are sufficiently strong to support the weight of the vessel and/or separator, whilst also being capable of providing the necessary adjustments in height to control the flow.

In some embodiments, the apparatus may further comprise a frame, the vessel and/or the separator, preferably the separator, being mounted to the frame, the vertical-positioning adjuster being connected to the frame such that the vertical-positioning adjuster can change the position relative to a vertical axis of the frame and so change the position relative to a vertical axis of the vessel and/or the separator during the course of the biotechnological process. The presence of a frame allows the vertical positioning adjuster to be connected indirectly to the separator i.e. via the frame. This can simplify the apparatus since modification of the separator for connection specifically to the vertical positioning adjuster can be avoided. The frame can also serve as a stable mounting point on or in which the separator is mounted. It will be understood that the frame may have any suitable form, for example it may comprise a platform upon which the separator stands, or it may comprise a base and/or top and side portions, within which the separator is housed. The same applies where the vessel instead of the separator is mounted to the frame.

In some embodiments, the separator may be a centrifuge. Preferably the centrifuge is configured to limit damage to the cells during the separation process. For example, the centrifuge may have a speed and/or g-force that is lower than those used for conventional pharma applications.

Where the separator is a centrifuge the centripetal pump of the centrifuge can supply the force needed to return the cells through the second conduit back to the vessel.

In some embodiments, the vessel, the first conduit and/or the second conduit comprise one or more sensors, such as a flow sensor or a pressure sensor. The vessel may comprise a pressure sensor. The first and second conduit may each comprise a flow sensor. The sensors enable the respective conditions to be monitored such that changes can be made. This could, for example, be to the height of the separator, where the flow of cells and culture medium from the vessel to the separator needs to be adjusted.

In embodiments in which the vessel, the first conduit and/or the second conduit comprise one or more sensors, the sensor, some of the sensors or all of the sensors may be connected to one or more controllers, the one or more controllers being configured to activate a response in the vertical-positioning adjuster, such that the position of the vessel and/or the separator, preferably the separator, relative to a vertical axis can be adjusted based on the measurements made by the sensor(s). Thus, the combination of the sensor or sensors with one or more controllers enables automation of the system. The sensor or sensor(s) can be used to measure parameters such as flow rate from the vessel to the separator and if the flow rate differs from the required flow rate, or falls outside of the target flow rate range the controller can activate the vertical-positioning adjuster and thereby raise or lower the vessel or the separator accordingly until the flow rate matches the required target or falls within the target range. Measurements can be made on an intermittent or continual basis and height adjustments made, preferably automatically, as required.

In some embodiments, the first conduit and/or the second conduit may have a variation in cross sectional area in the entire length of the conduit between the vessel and the separator of less than 25%. The first conduit and/or the second conduit may have a variation in cross sectional area in the entire length of the conduit between the vessel and the separator of less than 20%. The first conduit and/or the second conduit may have a variation in cross sectional area in the entire length of the conduit between the vessel and the separator of less than 15%. The first conduit and/or the second conduit may have a variation in cross sectional area in the entire length of the conduit between the vessel and the separator of less than 10%. The first conduit and/or the second conduit may have a variation in cross sectional area in the entire length of the conduit between the vessel and the separator of less than 5%. The first conduit and/or the second conduit may have an essentially constant cross-sectional area in the entire length of the conduit between the bioreactor and the separator. Preferably the aforementioned variations apply to both the first and second conduit. Points or regions within a flow path in which the cross section narrows typically result in the application of shear stress to passing cells. By having limited variation in cross sectional area along the length of the first and/or second conduit, the resulting application of shear stress is reduced, thereby reducing cell damage. An essentially constant cross section along the entire length of the first and second conduit between the vessel and the separator can significantly reduce the shear stress applied to cells between the vessel and the separator compared with conventional systems, such as those in which pinch valves or like act to control flow by limiting the cross-sectional area.

In some embodiments, the first conduit and/or the second conduit contain no components which reduce the cross-sectional area of the conduit(s), such as valves and pumps. Thus, the shear stress applied to cells as they travel between vessel and separator is significantly reduced compared to embodiments in which components such as pinch valves and pumps are present. This can in turn improve the overall yield.

In some embodiments, the second separator outlet is connected to a media rejuvenation device. Thus, culture medium removed in the separator can be regenerated. It may subsequently be returned to the vessel, reducing the overall amount of culture medium required. The connection may be direct, or via a conduit, or other intermediate component.

In some embodiments, the biotechnological process involves growth, maintenance, proliferation and/or differentiation of target cells and/or use of cells in the production of a target product such as protein(s). Thus, the apparatus can be beneficial both for processes which involve the growth or maintenance of cells, and also in the use of such cells for example in the production of products which may be, or may comprise, peptides, proteins or small molecules. The target product may be a product suitable for human and/or animal consumption.

The biotechnological process may involve culture of cells. Thus, it may involve growing and/or maintaining cells, such as the cell types discussed below, outside their natural environment, preferably in an environment which is under controlled conditions, and which is optimised for growth and/or maintenance of the cells.

The biotechnological process could be biomass fermentation in which the cells form at least part of the target product. The biotechnological process could be precision fermentation in which the cells are used to produce a target product, but where the cells are not normally part of the target product. The biotechnological process could involve growth of cells for further proliferation and/or differentiation and/or modification, for example formation of cell precursors for use in the formation of cultured meat.

In some embodiments, the cells may be eukaryotic cells, preferably eukaryotic cells derived from complex multicellular organisms, even more preferred eukaryotic cells from the mesodermal lineage. The apparatus can therefore be beneficial in a wide range of applications, including in pharma production processes and in the emerging new foods sector.

The cells may be bacteria, yeast, or fungus. The cells may be animal cells, mammalian cells, fish cells, insect cells or plant cells. Where the cells are mammalian cells, they may be muscle satellite cells, mesenchymal stem/stromal cells (MSCs), or fibro/adipogenic progenitors (FAPs). The invention can be considered particularly beneficial for those cells which are sensitive to shear stress.

According to a second aspect of the invention there is provided a method of controlling flow of cells and culture medium between a vessel and a separator, for example using the apparatus of the first aspect of the invention, the method comprising the steps of:

i) providing a vessel, the vessel being a bioreactor or a storage tank, the vessel comprising a vessel inlet and a vessel outlet, the vessel having a fill level and containing cells and culture medium, preferably the overpressure applied within the vessel is in the range 0 - 100 kPa,

ii) providing a separator, the separator comprising a separator inlet, a first separator outlet and a second separator outlet,

iii) providing a first conduit from the vessel outlet to the separator inlet through which cells and culture medium can pass,

iv) providing a second conduit from the first separator outlet to the vessel inlet through which cells and optionally culture medium can pass following a separation process,

v) providing a vertical-positioning adjuster configured to change the position of the vessel and/or the separator relative to a vertical axis during the course of the biotechnological process, preferably the vertical-positioning adjuster is configured to change the position of the separator relative to a vertical axis during the course of the biotechnological process,

vi) arranging the separator so that the separator inlet is at a height that is equal to or above the height of the fill level of the vessel,

vii) allowing cells and culture medium to travel from the vessel to the separator in the first conduit, and allowing cells and optionally culture medium to travel from the separator to the vessel in the second conduit following a separation process,

viii) monitoring the flow rate of cells and culture medium between the vessel and the separator and comparing the measured flow rate with a predetermined target value or range, and

ix) where the measured flow rate differs from the predetermined target or falls outside the predetermined target range, operating the vertical-positioning adjuster to change the position of the vessel and/or the separator, preferably the separator, relative to a vertical axis so that the measured flow rate matches the predetermined target or falls within the predetermined range.

It will be understood that the reference to overpressure is in comparison with the surroundings within which the vessel is positioned, which are typically at atmospheric pressure.

The use of the vertical-positioning adjuster configured to change the position of the vessel and/or the separator relative to a vertical axis during the course of the biotechnological process provides a means by which the flow of cells and culture medium between the vessel and the separator can be controlled. As a result, the reliance on components such as pumps and/or pinch valves which control flow via a reduction in cross sectional area of the flow path is reduced or even obviated. This is beneficial as the shear stress to which the cells are exposed as a result of use of components such as pinch valves and pumps can be reduced, thereby reducing damage to cells and in turn providing greater yield by use of the method. The second aspect of the invention therefore provides a method of controlling flow of cells and culture medium between a vessel and a separator, which facilitates perfusion in a way that limits cell damage compared with conventional systems.

Preferably steps viii) and ix) are repeated throughout the course of the biotechnological process, such as wherein step viii) is conducted continually and step ix) is conducted as required. Thus, the flow between the vessel and the separator is monitored and the vessel and/or separator, preferably separator, is raised or lowered as required when changes to the flow are required.

In some embodiments, in step ix) the vertical positioning adjuster is operated to change the position of the vessel and/or the separator, preferably the separator, relative to a vertical axis by an amount of 50 cm or less, preferably by an amount of 25 cm or less, more preferred by an amount of 10 cm or less. Thus, relatively small changes in the vertical position of the vessel and/or separator, preferably the separator, can be used to fine tune the flow rate between the vessel and the separator. It will be understood that the vertical positioning adjuster may also be used in step vi) arranging the separator so that the separator inlet is at a height that is equal to or above the height of the fill level of the vessel, but in this stage the range of movement will typically be much larger than the fine tuning adjustment in order to move the separator from a starting point which will typically be ground level to a position at which the separator inlet is at a height that is equal to or above the height of the fill level of the vessel.

The embodiments discussed above in connection with the first aspect of the invention may be utilised in the method of the second aspect of the invention.

### Brief Description of Drawings

Embodiments of the invention will be described in the following detailed description with reference to the drawings in which:

>**Figure 1** shows a simplified schematic view of an apparatus according to the invention in which the separator can be raised and lowered,
>**Figure 2** shows a more detailed representation of the apparatus according to figure 1, and
>**Figure 3** shows a visual representation of the vertical axis in relation to which the vertical-positioning adjuster causes a change in position.

### Detailed Description of Embodiments

It will be understood that in the context of the present application culture medium is any liquid containing the necessary metabolites for a target cell or cells to grow, remain viable, proliferate, differentiate and/or produce one or more target products such as proteins.

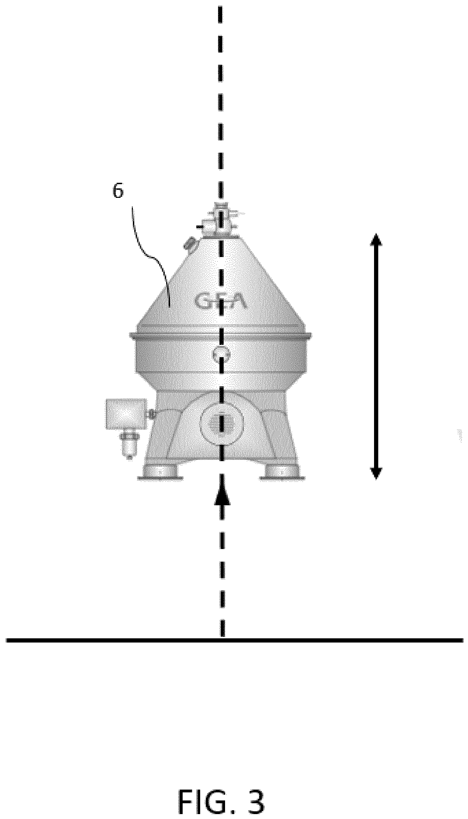

Likewise, it will be understood that a vertical-positioning adjuster is a component which changes the position of the vessel and/or the separator relative to a vertical axis during the course of the biotechnological process. In other words, the height at which the vessel and/or separator is positioned is raised or lowered accordingly. The vertical axis is perpendicular to the horizontal plane of the ground. This is represented schematically in Fig. 3. Bioreactors are often cylindrical, and in instances where a cylindrical bioreactor is used, the axis of the cylinder is expected to be parallel with the vertical axis.

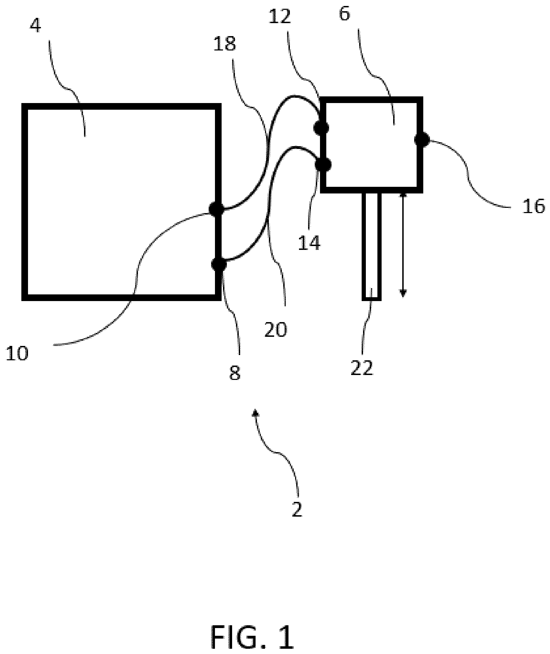

Referring to Fig. 1, a schematic view of an apparatus 2 according to the invention is shown. The apparatus 2 comprises a vessel 4. In this embodiment shown the vessel 4 is a bioreactor. The bioreactor may have any suitable volume, such as $0.05\ m^3$ - $25\ m^3$, as typically used at production scales, or a smaller volume below $0.05\ m^3$ for other applications, such a pilot applications or lab applications. Alternatively, the vessel 4 could be a storage tank. The vessel 4 comprises a vessel inlet 8 and a vessel outlet 10. For simplicity these components are represented schematically but it will be understood that they may have any suitable form as known to the skilled person.

The apparatus 2 also comprises a separator 6. In the embodiment shown the separator 6 is a centrifuge. The separator 6 comprises a separator inlet 12, a first separator outlet 14 and a second separator outlet 16. Again, for simplicity these components are represented schematically but may have any suitable form as known to the skilled person. An example of a suitable centrifuge includes those in the GEA kytero® range.

The vessel inlet 8, vessel outlet 10, separator inlet 12, the first separator outlet 14 and/or second separator outlet may be provided with a valve or other means to prevent flow. These can be used to ensure that cells and culture medium only leave or enter the vessel 4 or separator 6 when required.

The apparatus 2 further comprises a first conduit 18 from the vessel outlet 10 to the separator inlet 12 through which cells and culture medium can pass. The form of the first conduit can be selected according to the process with which the apparatus is to be used. For example, parameters such as the length of the conduit, cross-sectional shape and area of the conduit and material(s) from which it is made can be selected accordingly. The first conduit will in general be flexible and inert to the cells and culture medium which travel within.

The apparatus 2 comprises a second conduit 20 from the first separator outlet 14 to the vessel inlet 8 through which cells and optionally culture medium can pass following a separation process. The separation process will in general separate the cells from the culture medium, with the cells passing back to the vessel 4 from the separator 6. However, the separation need not necessarily result in complete separation of cells from culture medium and thus the second conduit 20 may additionally contain culture medium in addition to cells. As for the first conduit 18, the form of the second conduit 20 can be selected according to the process or processes with which the apparatus is to be used. For example, parameters such as the length of the conduit, cross-sectional shape and area of the conduit and material(s) from which it is made can be selected accordingly. The second conduit 20 will in general be flexible and inert to the cells and culture medium which travel within.

In general, the first and second conduit may have the same length as each other, the same cross-sectional shape and/or the same cross-sectional area as each other. Likewise, the first and second conduits may be made of the same material(s) as each other. However, in some embodiments it may be advantageous if the first and second conduits are not identical. For example, if the positioning of the respective inlets and outlets means that it is beneficial for the conduits to be of differing lengths. In general, the first and second conduit will be configured to account for the relative movement between the vessel 4 and the separator 6. That could, for example, include ensuring the length of the conduit exceeds the separation between the vessel 4 and the separator 6 and includes enough excess length to accommodate the raising/lowering of the vessel 4 and/or separator 6.

The apparatus 2 further comprises a vertical-positioning adjuster 22. The vertical positioning adjuster 22 is configured to change the position of the vessel 4 and/or the separator 6 relative to a vertical axis during the course of the biotechnological process. In the embodiment shown the vertical-positioning adjuster 22 is configured to change the position of the separator 6 relative to a vertical axis during the course of the biotechnological process. For simplicity, the vertical-positioning adjuster is represented schematically. However, it will be understood that it may comprise an electric or hydraulic position changing mechanism. This could, for example be an electric or hydraulic lift which is suitable to support the weight of the component to be moved, which in the example of Fig. 1 is the separator 6. Whilst a single vertical positioning adjuster 22 could be configured to independently raise and lower the vessel 4 and the separator 6, preferably it will act on just one component, typically the separator 6. The vertical-positioning adjuster 22 need not be limited to only upwards and downwards movements. It may additionally provide an off-vertical movement, or even horizontal motion in addition to the upwards and downwards motion.

The apparatus may further comprise a frame (not shown in the figures). The vessel 4 and/or the separator 6 may be mounted to the frame, the vertical-positioning adjuster 22 being connected to the frame such that the vertical-positioning adjuster 22 can change the position relative to a vertical axis of the frame and so change the position relative to a vertical axis of the vessel 4 and/or the separator 6 during the course of the biotechnological process. In the embodiment represented by Fig. 1 the vertical-positioning adjuster 22 is configured to change the position of the separator 6 relative to a vertical axis during the course of the biotechnological process. Thus, in this embodiment, the separator 6 would be mounted to the frame. The vertical-positioning adjuster 22 would move the frame, and in turn move the separator 6. It will be understood that the frame may have any suitable form, for example it may comprise a platform upon which the separator stands, or it may comprise a base and/or top and side portions, within which the separator is housed. In the case of a platform-type frame, the separator may sit on the platform, preferably secured via suitable anchoring means such as bolts. The platform can then rest on top of the vertical-positioning adjuster 22, preferably with further suitable securing means such as bolts or other connectors. As the vertical-positioning adjuster 22 is activated to raise or lower, it raises or lowers the platform, which in turn results in the raising or lowering of the separator 6.

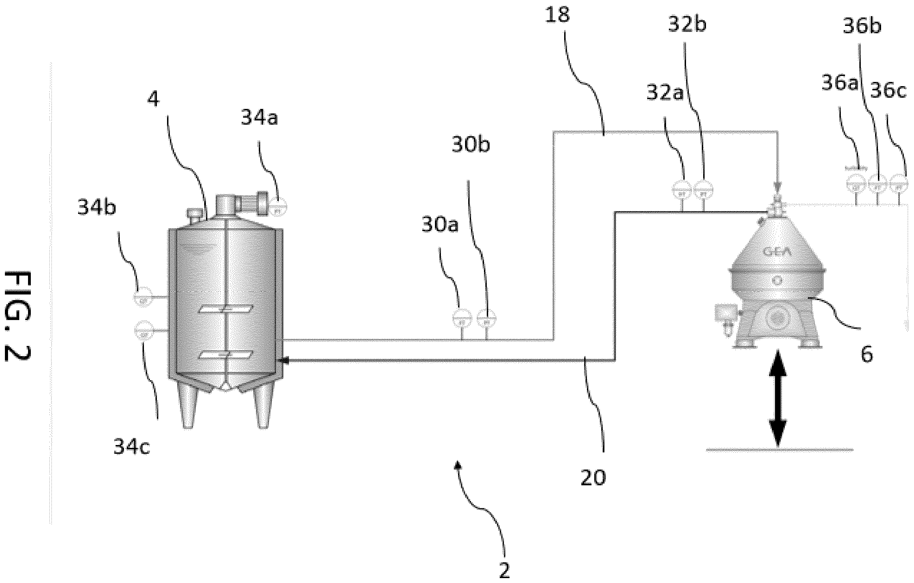

Fig. 2 is a further schematic representation of the apparatus shown in Fig. 1 in which some additional details of the apparatus are shown. In particular, some of the sensor possibilities are shown. It will be understood that it is not expected that all of the sensors need to be present in a single system, but they are shown for representative purposes. As shown in Fig.2 there is first conduit flow sensor 30a, first conduit pressure sensor 30b, second conduit flow sensor 32a, second conduit pressure sensor 32b, vessel pressure sensor 34a, vessel metabolite concentration sensor 34b, vessel cell mass/density sensor 34c, turbidity sensor 36a, additional flow sensor 36b and additional pressure sensor 36c. A preferable configuration includes first conduit flow sensor 30a, second conduit flow sensor 32a. Examples of suitable sensors include ultrasonic clamp on sensors for flexible tubing of the Leviflow® LFSC-iX family by Levitronix and single-use ultrasonic inline sensors of the Leviflow® LFS-SU family by Levitronix. In addition to the two flow sensors a preferable configuration also includes vessel pressure sensor 34a.

Sensors such as first conduit flow sensor 30a and second conduit flow sensor 32a could be connected to one or more controllers (not shown), the one or more controllers being configured to activate a response in the vertical-positioning adjuster 22, such that the position of the vessel 4 and/or the separator 6, preferably the separator 6, relative to a vertical axis can be adjusted based on the measurements made by the sensor(s). This enables automation of the process, where the controller(s) can move the separator 6 as required without requiring user input. Alternatively, a user could monitor the readings from the sensors and activate the necessary change in height of the vertical-positioning adjuster 22.

For simplicity, in the figures no onward connection of the second separator outlet 16 is shown. The second separator outlet 16 may be connected to a media rejuvenation device (not shown). In the way, spent culture medium that is extracted in the separator can be rejuvenated for further use, and can thus be recycled back to the vessel 4 from the media rejuvenation device. In some embodiments, it may not be effective or cost-efficient use media rejuvenation, in which case the second separator outlet 16 may be connected an alternative further processing means, or a drain. As represented in Fig. 2, turbidity sensor 36a, additional flow sensor 36b and/or additional pressure sensor 36c may be used to monitor the output from the second separator outlet 16, for example on the way to such a media rejuvenation device or an alternative connection.

It may be beneficial, in general, to use a sensor or offline sampling means to determine cell concentration, particularly viable cell concentration, in the vessel or in the one or more of the conduits. This could be achieved for example by assessing turbidity / optical density or permittivity.

Further, a sensor or offline sampling means could be beneficial to determine concentration of metabolites such as but not limited to glucose, lactate, glutamine and/or ammonia in the vessel or in one or more of the conduits.

In the embodiments represented by Fig. 1 and Fig. 2 the first conduit 18 and the second conduit 20 both have an essentially constant cross-sectional area in the entire length of the conduit between the bioreactor and the separator. Further, there are no pumps, pinch valves, or other components which reduce the cross-sectional area of the conduit. Thus, the change of height of the separator 6 serves as the mechanism to control the flow of cells and culture medium to the separator from the vessel 4. Thus, in the embodiment shown no other flow controlling means are required, eliminating the shear stress applied to cells by such flow controlling means. In other embodiments, other flow controlling means such as pumps and/or pinch valves may be present, but the reliance on them is reduced since some of the flow is controlled by the change of height of the separator 6 or vessel 4. Thus, in these embodiments cell damage will still be reduced compared with conventional systems.

The apparatus shown may be used with a range of biotechnological processes and in particular those which involve growth, maintenance, proliferation or differentiation of target cells and/or use of cells in the production of a target product such as protein(s). The cells may be eukaryotic cells, preferably eukaryotic cells derived from complex multicellular organisms, even more preferred eukaryotic cells from the mesodermal lineage.

Regardless of the specific biotechnological process, the vessel 4, such as the bioreactor will be provided with cells and culture medium and will be filled to a particular level, referred to as the fill level. It will be understood that vessels such as bioreactors typically have a nominal fill level which is the fill level with which they are designed or intended to operate. However, the actual fill level and the nominal fill level need not be the same. Likewise, the actual fill level may vary over time as matter is consumed, spend medium is removed or as new culture medium is added.

As a starting point the separator 6 will be arranged so that the separator inlet 12 is at a height that is equal to or above the height of the fill level of the vessel 4. If the vessel 4 already contains cells and culture medium the fill level will be known. If the separator 6 is to be arranged at or above the fill level before the vessel 4 is filled, the fill level will be the level to which the vessel is to be filled when the cells and culture medium are added.

During the perfusion process cells and culture medium are allowed to travel from the vessel 4 to the separator 6 in the first conduit 18. In the separator 6, the cells and culture medium are separated, with the cells and optionally some remaining culture medium being allowed to travel from the separator 6 to the vessel 4 in the second conduit 20 following the separation process.

The flow rate of cells and culture medium between the vessel 4 and the separator 6 can be monitored with the sensors discussed above and measurements compared either manually or preferably automatically with a predetermined target value or range. Where the measured flow rate differs from the predetermined target or falls outside the predetermined target range, the vessel 4 and/or the separator 6 can be raised or lowered to adjust the flow rate accordingly. In the embodiments represented by the figures it is the separator 6 only that is raised and lowered by the vertical-positioning adjuster 22.

At or near the start of the process separator 6 will be arranged so that the separator inlet 12 is at a height that is equal to or above the height of the fill level of the vessel 4. Preferably, to start with, the separator 6 will be arranged so that the separator inlet 12 is at the same height as the fill level of the vessel 4. The aim is to have hydrostatic balance between the vessel 4 and the separator 6. Some adjustment may be required to account for the pressure within the vessel 4 in comparison with the pressure loss across the first conduit 18.

The flow rate at the separator inlet 12 depends on Bernoulli's equation and any change in height of the vessel or separator will change the flow velocity:

$$p_{O,B} + p_{h,B} + p_{v,B} = p_{O,S} + p_{h,S} + p_{v,S} + p_L$$

where:

$p_{O,B}$ is the pressure applied to the vessel;
$p_{h,B}$ is the hydrostatic pressure of the liquid in the vessel;
$p_{v,B}$ is the dynamic pressure resulting from motion of the fluid within the vessel;
$p_{O,S}$ is the applied pressure of the separator;
$p_{h,S}$ is the hydrostatic pressure of the liquid in the separator;
$p_{v,S}$ is the dynamic pressure resulting from motion of the fluid within the separator; and
$p_L$ is the pressure loss across the conduit from vessel to separator.

The formula can be simplified, assuming that the vessel contents have no velocity in the vessel and that there is no applied pressure in the separator. Under these assumptions the formula simplifies to:

$$p_{O,B} + p_{h,B} = p_{h,S} + p_{v,S} + p_L$$

It will be understood that if the applied pressure within the vessel and the fill level of the vessel are constant, the left side of the above equation will be constant. Thus, there is a direct correlation between height and flow speed at the separator, meaning that lifting the separator will reduce the flow speed while lowering will increase the flow speed. Therefore, the separator can be moved up and down to control the flow rate to the separator.

Where the separator is a centrifuge the centripetal pump can supply the force needed to return the cells through the second conduit back to the vessel.

In some embodiments the liquid level in the vessel might change over time e.g. in fed-batch processes, and the vertical positioning adjuster can therefore be configured to move the position of the vessel or separator to accommodate this changing level. A changing fill level means a non-constant $p_{h,B}$ value and thus keeping the same flow speed will require a height change of the separator.

The following factors will generally need to be taken into consideration when considering the target flow rate between vessel and separator:

- Cell density in the vessel: higher cell density results in higher usage of nutrients, resulting in higher flow rates through the first conduit to the separator, and corresponding impact on flows leaving the separator;
- Concentration of one or more nutrients in the vessel: greater availability of nutrients can result in higher cell densities, leading to the impacts discussed above for cell density;
- Concentration of one or more metabolites: higher media exchange rate needed for high metabolite concentrations, meaning greater flow rate to separator.

Thus, these factors can impact upon the height adjustments needed to the vessel and/or the separator, preferably the separator, in order to give the desired flow. In addition, changes of the liquid level within the vessel (hydrostatic pressure) and changes to vessel overpressure will need to be accounted for.

The flow rate from the vessel to the separator can be fixed or can vary during the course of a process.

Thus, the apparatus and method described herein provide means for perfusion which can reduce cell damage compared with conventional systems. In the perfusion process, spent culture medium and/or culture medium enriched with inhibiting metabolites is removed and according to the invention this is achieved whilst limiting damage to the cells. The reduced cell damage compared with conventional systems is advantageous, as reduced cell loss and therefore increased yields can be achieved.

## List of Reference Numerals

2 -     apparatus for use in a biotechnological process
4 -     vessel
6 -     separator

8 -     vessel inlet
10 -    vessel outlet
12 -    separator inlet
14 -    first separator outlet
16 -    second separator outlet
18-     first conduit
20-     second conduit
22-     vertical positioning adjuster

30a -   first conduit flow sensor
30b -   first conduit pressure sensor
32a -   second conduit flow sensor
32b -   second conduit pressure sensor
34a -   vessel pressure sensor
34b -   vessel metabolite concentration sensor
34c-    vessel cell mass/density sensor
36a -   turbidity sensor
36b -   additional flow sensor
36c -   additional pressure sensor

## Claims

1. An apparatus for use in a biotechnological process, the apparatus comprising:

   a vessel, the vessel being a bioreactor or storage tank, the vessel comprising a vessel inlet and a vessel outlet,
   a separator, the separator comprising a separator inlet, a first separator outlet and a second separator outlet,
   there being a first conduit from the vessel outlet to the separator inlet through which cells and culture medium can pass,
   there being a second conduit from the first separator outlet to the vessel inlet through which cells and optionally culture medium can pass following a separation process,
   **characterized in that** the apparatus further comprises a vertical-positioning adjuster configured to change the position of the vessel and/or the separator relative to a vertical axis during the course of the biotechnological process, preferably the vertical-positioning adjuster is configured to change the position of the separator relative to a vertical axis during the course of the biotechnological process.

2. An apparatus according to claim 1, wherein the vertical-positioning adjuster comprises an electric or hydraulic position changing mechanism.

3. An apparatus according to claim 1 or claim 2, wherein apparatus further comprises a frame, the vessel and/or the separator, preferably the separator, being mounted to the frame, the vertical-positioning adjuster being connected to the frame such that the vertical-positioning adjuster can change the position relative to a vertical axis of the frame and so change the position relative to a vertical axis of the vessel and/or the separator during the course of the biotechnological process.

4. An apparatus according to any preceding claim, wherein the separator is a centrifuge.

5. An apparatus according to any preceding claim, wherein the vessel and/or the first conduit and/or the second conduit comprise one or more sensors, such as a flow sensor or a pressure sensor.

6. An apparatus according to claim 5, wherein the sensor, some of the sensors or all of the sensors are connected to one or more controllers, the one or more controllers being configured to activate a response in the vertical-positioning adjuster, such that the position of the vessel and/or the separator, preferably the separator, relative to a vertical axis can be adjusted based on the measurements made by the sensor(s).

7. An apparatus according to any preceding claim, wherein the first conduit and/or the second conduit has a variation in cross sectional area in the entire length of the conduit between the vessel and the separator of less than 25%, preferably the first conduit and/or the second conduit has a variation in cross sectional area in the entire length of the conduit between the vessel and the separator of less than 10%, even more preferred the first conduit and/or the second conduit has as an essentially constant cross sectional area in the entire length of the conduit between the bioreactor and the separator.

8. An apparatus according to any preceding claim, wherein the first conduit and/or the second conduit contain no components which reduce the cross-sectional area of the conduit(s), such as valves and pumps.

9. An apparatus according to any preceding claim, wherein the second separator outlet is connected to a media rejuvenation device.

10. An apparatus according to any preceding claim, wherein the biotechnological process involves growth, maintenance, proliferation and/or differentiation of target cells and/or use of cells in the production of a target product.

11. An apparatus according to any preceding claim, wherein the cells are eukaryotic cells, preferably eukaryotic cells derived from complex multicellular organisms, even more preferred eukaryotic cells from the mesodermal lineage.

12. A method of controlling flow of cells and culture medium between a vessel and a separator, for example using the apparatus of any of claims 1 to 11, the method comprising the steps of:

i) providing a vessel, the vessel being a bioreactor or a storage tank, the vessel comprising a vessel inlet and a vessel outlet, the vessel having a fill level and containing cells and culture medium, preferably the overpressure applied within the vessel is in the range 0 - 100 kPa,
ii) providing a separator, the separator comprising a separator inlet, a first separator outlet and a second separator outlet,
iii) providing a first conduit from the vessel outlet to the separator inlet through which cells and culture medium can pass,
iv) providing a second conduit from the first separator outlet to the vessel inlet through which cells and optionally culture medium can pass following a separation process,
v) providing a vertical-positioning adjuster configured to change the position of the vessel and/or the separator relative to a vertical axis during the course of the biotechnological process, preferably the vertical-positioning adjuster is configured to change the position of the separator relative to a vertical axis during the course of the biotechnological process,
vi) arranging the separator so that the separator inlet is at a height that is equal to or above the height of the fill level of the vessel,
vii) allowing cells and culture medium to travel from the vessel to the separator in the first conduit, and allowing cells and optionally culture medium to travel from the separator to the vessel in the second conduit following a separation process,
viii) monitoring the flow rate of cells and culture medium between the vessel and the separator and comparing the measured flow rate with a predetermined target value or range, and
ix) where the measured flow rate differs from the predetermined target or falls outside the predetermined target range, operating the vertical-positioning adjuster to change the position of the vessel and/or the separator, preferably the separator, relative to a vertical axis so that the measured flow rate matches the predetermined target or falls within the predetermined range.

13. A method according to claim 12, wherein steps viii) and ix) are repeated throughout the course of the biotechnological process, such as wherein step viii) is conducted continually and step ix) is conducted as required.

14. A method according to claims 12 or 13, wherein in step ix) the vertical positioning adjuster is operated to change the position of the vessel and/or the separator, preferably the separator, relative to a vertical axis by an amount of 50 cm or less, preferably by an amount of 10 cm or less.

FIG. 1

FIG. 2

6

GFA

FIG. 3

EP 4 763 965 A1

Europäisches
Patentamt
European
Patent Office
Office européen
des brevets

**EUROPEAN SEARCH REPORT**

Application Number

EP 24 22 2448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 201 971 840 U (ZHENGZHOU VIRI BIOTECHNOLOGY CO LTD) 14 September 2011 (2011-09-14) | 1-5,7-11 | INV. C12M1/00 C12M1/34 |
| A | * the whole document * | 6,12-14 | |
| Y | US 2009/104698 A1 (DUTRA TIMOTHY F [US]) 23 April 2009 (2009-04-23) | 1-5,7-11 | |
| A | * paragraph [0037] - paragraph [0039] * * paragraph [0050] - paragraph [0051] * * paragraph [0060] - paragraph [0066] * * figures 1,4,5 * | 6,12-14 | |
| Y | US 2011/217690 A1 (NIAZI SARFARAZ K [US]) 8 September 2011 (2011-09-08) | 1-5,7-11 | |
| A | * paragraph [0016] * * paragraph [0036] - paragraph [0038] * * figures 4,5 * | 6,12-14 | |
| A | US 2012/100576 A1 (GOLETZ STEFFEN [DE] ET AL) 26 April 2012 (2012-04-26) * claims 1-20 * | 1-14 | |
| A | CN 109 321 437 B (2ND XIANGYA HOSPITAL CENTRAL SOUTH UNIV) 28 September 2021 (2021-09-28) * the whole document * | 1-14 | TECHNICAL FIELDS SEARCHED (IPC) C12M |
| A | US 2018/072978 A1 (SATOU KANETOMO [JP] ET AL) 15 March 2018 (2018-03-15) * paragraph [0078] * * paragraph [0083] - paragraph [0085] * * figure 3 * | 1-14 | |

The present search report has been drawn up for all claims

| Place of search | Date of completion of the search | Examiner |
|---|---|---|
| Berlin | 28 May 2025 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

14

## ANNEX TO THE EUROPEAN SEARCH REPORT
## ON EUROPEAN PATENT APPLICATION NO.

EP 24 22 2448

28-05-2025

| Patent document cited in search report | | Publication date | Patent family member(s) | | Publication date |
|---|---|---|---|---|---|
| CN 201971840 | U | 14-09-2011 | NONE | | |
| US 2009104698 | A1 | 23-04-2009 | US 7476541 B1 | | 13-01-2009 |
| | | | US 2009104698 A1 | | 23-04-2009 |
| US 2011217690 | A1 | 08-09-2011 | US 2011217690 A1 | | 08-09-2011 |
| | | | US 2015275318 A1 | | 01-10-2015 |
| US 2012100576 | A1 | 26-04-2012 | EP 2451935 A2 | | 16-05-2012 |
| | | | US 2012100576 A1 | | 26-04-2012 |
| | | | WO 2011003615 A2 | | 13-01-2011 |
| CN 109321437 | B | 28-09-2021 | NONE | | |
| US 2018072978 | A1 | 15-03-2018 | CA 2979789 A1 | | 29-09-2016 |
| | | | CN 107406819 A | | 28-11-2017 |
| | | | EP 3272853 A1 | | 24-01-2018 |
| | | | EP 3656845 A1 | | 27-05-2020 |
| | | | JP 6706612 B2 | | 10-06-2020 |
| | | | JP WO2016152697 A1 | | 18-01-2018 |
| | | | US 2018072978 A1 | | 15-03-2018 |
| | | | WO 2016152697 A1 | | 29-09-2016 |

EPO FORM P0459

## REFERENCES CITED IN THE DESCRIPTION

**Patent documents cited in the description**

- EP 2020433 A
- WO 2005042768 A